# NEUE EUROPÄISCHE PATENTSCHRIFT

(11) **EP 0 173 242 B2**
(45) Veröffentlichungstag und Bekanntmachung des Hinweises auf die Entscheidung über den Einspruch: **24.07.1996**
(45) Hinweis auf die Patenterteilung: 18.03.1992
(21) Anmeldenummer: 85110499.2
(22) Anmeldetag: 21.08.1985
(51) Int. Cl.: A61K 35/16, C12P 21/02

(54) **Verfahren zur Herstellung einer pasteurisierten, isoagglutininfreien Faktor VIII Präparation aus einem Kryopräzipitat**
Process for the production of a pasteurized isoagglutinin-free Faktor VIII preparation from a cryoprecipitate.
Procédé de préparation à partir d un kryoprécipité d une préparation pasteurisée de Facteur VIII exempte d isoagglutinine

(30) Priorität: 31.08.1984 DE 3432083
(43) Veröffentlichungstag der Anmeldung: 05.03.1986
(73) Patentinhaber: BEHRINGWERKE Aktiengesellschaft, 35001 Marburg (DE)
(72) Erfinder: Heimburger, Norbert, Prof. Dr., D-3550 Marburg 1 (DE); Wormsbächer, Wilfried, D-3575 Kirchhain-Niederwald (DE); Kumpe, Gerhardt, D-3552 Wetter (DE)

(56) Entgegenhaltungen:
- EP-A- 0 083 483
- EP-A- 0 106 269
- EP-A- 0 144 957
- DE-A- 2 635 894
- DE-A- 2 653 534
- DE-A- 2 916 711
- DE-A- 3 237 512
- DE-A- 3 237 512
- THROMBOSIS RESEARCH, Band 1, Nr. 2, 1972, Seiten 197-200, US; R.L. LUNDBLAD et al.: "The effect of dextrose on chromatography of antihemophilic factor (factor VIII)"
- British Journal of Haematologie, 1973 (43) 669-674 Austen.
- Römpps Chemielexikon, achte Auflage, S. 642.
- XVIII International congress of the World Federation of Hemophilia, Madrid May 26-31 1988, "RESPONSE AND HALF-LIFE OF THE HIGH CONCENTRATE FACTOR VIII C HS COMPARED WITHFACTOR VIII HS IN A DOUBLE BLIND STUDY." Schimpf Kl an Schumacher K.
- Faure et al., J. of Chromatographie, 257 (1983) 387-391
- Heimburger et al., Arzneim.-Forschung 31(1), Nr. 4 (1981), S. 619-622

## Beschreibung

Die Erfindung betrifft ein

Verfahren zur Herstellung einer Faktor VIII-Präparation, dadurch gekennzeichnet, daß eine pasteurisierte Faktor VIII enthaltende Lösung aus einem Kryopräzipitat aus Humanplasma in Gegenwart eines Kohlenhydrats und einer Aminosäure mit einem DEAE-, QAE- oder Ecteola-gruppentragenden Austauscher auf der Basis von Cellulose, Sephadex® oder Sepharose® bei einem pH von 5 - 6,5 behandelt wird, der Austauscher gewaschen wird und der F VIII mit chaotropen Lösungen, vorzugsweise mit einer Lösung von CaCl₂ eluiert wird und ein

Verfahren zur Herstellung einer Faktor VIII-Präparation, dadurch gekennzeichnet, daß Kryopräzipitat unter Zugabe von Saccharose und Glycin, vorzugsweise 35 - 60 g Saccharose/100 ml und 1-3 mol Glycin/l gelöst wird, daß Calciumionen, vorzugsweise 1-20 mmol/l, zugesetzt werden, daß gegebenenfalls pasteurisiert, abgekühlt und verdünnt wird, und daß bei pH 5 - 6,5 der F VIII:C an einen DEAE-, QAE- oder Ecteola-gruppentragenden Austauscher auf der Basis von Cellulose, Sephadex® oder Sepharose® adsorbiert und das Adsorbens gewaschen wird, bis es frei von Fremdproteinen, speziell Isoagglutinen, ist, und daß der F VIII:C durch Elution mit einer gepufferten konzentrierten chaotropen Salzlösung, vorzugsweise mit einer Lösung von CaCl₂ eluiert, dialysiert und lyophilisiert wird.

Die Blutgerinnung ist ein komplexer Vorgang, der über mehrere Reaktionsstufen abläuft und an dem mindestens 13 Gerinnungsfaktoren (F) beteiligt sind, die mit römischen Ziffern gekennzeichnet werden. Bei den Gerinnungsfaktoren handelt es sich vorwiegend um Proteine und speziell solche, die mit den Eigenschaften von Proteasen oder Akzelleratoren ausgestattet sind. Das einzig wirkliche Substrat ist Fibrinogen, das bei Verletzungen vom Thrombin in seine unlösliche Form, das Fibrin, überführt wird, das den primären Wundverschluß bildet. Wenn einer der 13 Gerinnungsfaktoren fehlt, bleibt die Bildung von Thrombin und Fibrin aus; die Folge sind Blutungen. Ein solcher Fall liegt bei der Hämophilie A vor, der am meisten verbreiteten Blutungskrankheit, gekennzeichnet durch einen Mangel an Faktor VIII. Die Hämophilie A und auch B (F IX-Mangel) kann nur durch Substitution des fehlenden Faktors wirksam therapiert werden.

Die Gewinnung von F VIII aus Humanplasma mit guter Ausbeute und hoher Reinheit, wie sie speziell für die Selbstbehandlung von Patienten erforderlich ist, stellt immer noch ein nicht optimal gelöstes Problem dar. Das trifft vor allem auf pasteurisierte F VIII-Konzentrate zu, welche die übliche Handelsware zunehmend verdrängten, weil damit das Risiko einer Hepatitisübertragung ausgeschaltet werden konnte.

Die Isolierung eines hochgereinigten F VIII mit guter Ausbeute wird vor allem dadurch erschwert, daß der F VIII zwar im Kryopräzipitat angereichert aber mit Fibrinogen und Fibronectin, zwei relativ hochmolekularen und schwerlöslichen Proteinen mit ähnlichen physikalischchemischen Eigenschaften wie der F VIII, vergesellschaftet ist.

Ein Faktor VIII-Konzentrat sollte so rein wie nur möglich sein, also frei von unerwünschten anderen Proteinen und speziell von Immunglobulinen einschließslich der Isoagglutinine; denn es gibt Hinweise, daß die Zufuhr von unspezifischen Proteinen zu einer Überforderung des retikuloendothelialen Systems (RES) führt und zu einer Beeinträchtigung der Immunabwehr, gekennzeichnet durch eine Änderung in der Zusammensetzung der Lymphozyten-Population und der Immunglobuline. Dies gewinnt an Bedeutung in Verbindung mit der Tatsache, daß Hämophile lebenslang mit solchen F VIII-Konzentraten behandelt werden müssen. Daraus resultiert die Forderung nach einem nativen, hochgereinigten pasteurisierten F VIII-Konzentrat, d.h. einem Produkt, das eine Übertragung von Hepatitisviren und anderem infektiösen Material und eine Sensibilisierung gegen Alloantigene ausschließt.

Ein solches eine Übertragung von Hepatitis ausschließendes und isoagglutininfreies Präparat und ein Verfahren zu seiner Herstellung sind Gegenstand dieser Erfindung.

Es wurde gefunden, daß eine F VIII-haltige Lösung, die praktisch frei von Faktoren des Prothrombinkomplexes ist (F II, VII, ZX und X), in an sich bekannter Weise nach Zusatz von Stabilisatoren zum Schutze gegen eine thermische Inaktivierung pasteurisiert, die erhitzte Lösung im pH-Bereich von 5-6,5 mit einen Anionenaustauscher behandelt, der adsorbierte F VIII von Begleitproteinen, besonders Fibrinogen, Fibronectin und Immunglobulinen einschließlich Isoagglutininen, freigewaschen, mit einer konzentrierten Lösung von Na-, K- oder Ca-Salzen mit einem Halogen eluiert und beispielsweise durch Präzipitation aus dem Eluat gewonnen werden kann.

Ecteola-Cellulose und ein QAE-(Quaternary Amino Ethyl-) gruppentragender Anionenaustauscher wurden bereits für die F VIII-Reinigung verwendet (van Creveld, S. et al., Thromb.Diath.Haem. (1961) VI, No.2/3, 282 und Baugh, R. et al., Bioch.Biophys.Acta (1974) 371, 360). Eine Übertragung in den Technikums- oder Produktionsmaßstab war jedoch nicht möglich. Lediglich zur Hochreinigung von vorfraktioniertem Material konnte Ionenaustauscherchromatographie eingesetzt werden (Fay, Ph.J. et al., Proc.NatI.Acad.Sci. (1982) 79, 7200).
Die Adsorption mit hoher Spezifität erfolgt nur bei einem pH-Wert um 5,5; da aber fallen die meisten der im Kryopräzipitat enthaltenen Proteine, vor allem das Humanfibrinogen, bereits aus und speziell beim Kontakt mit dem Adsorbens in der Säule. Zudem sind nach den zitierten Arbeiten relativ große Mengen Adsorbens erforderlich. Das wiederum scheint einen enormen Einfluß auf die Ausbeute gehabt zu haben, die minimal war, bedingt offenbar durch eine unspezifische Adsorption des F VIII - seine physiologische Aufgabe ist ja, an unphysiologischen Oberflächen zu adhärieren - an die großen Adsorbensmengen. Es war auch gefunden worden, daß das an QAE-Austauscher hochgereinigte Material schnell an Aktivität verlor; daher wurden Anionenaustauscher für die F VIII-Produktion nicht verwendet.

Es wurde jedoch nun überraschenderweise gefunden, daß eine Chromatographie an basischen Ionenaustauschern durchaus für die Produktion von F VIII-Präparationen geeignet ist. Es war überraschend, daß ausgehend von einem pasteurisierten Kryopräzipitat in einem Schritt mittels einer Anionenaustauscherchromatographie eine hochgereinigte F VIII-Präparation gewonnen werden kann.

Gegenstand der Erfindung ist daher ein Verfahren zur Herstellung einer Faktor VIII-Präparation, in dem eine Faktor VIII-enthaltende Lösung aus einem Kryopräzipitat in Gegenwart eines Kohlenhydrats und einer Aminosäure pasteurisiert und gereinigt wird, dadurch gekennzeichnet, daß die Lösung mit einem DEAE-, QAE- oder Ecteola-gruppentragenden Austauscher auf der Basis von Cellulose, Sephadex^{R} oder Sepharose^{R} bei-einem pH-Wert von 5 - 6,5 behandelt wird, der Austauscher gewaschen wird und der F VIII mit chaotropen Lösungen, vorzugsweise mit einer Lösung von CaCl₂ eluiert wird.

Als Ausgangsmaterial kommt ein z.B. nach Pool et al., Nature (1964) 203, 312 gewonnenes Kryopräzipität in Betracht.

Als Stabilisatoren zum Schutz gegen eine thermische Inaktivierung des F VIII während der Pasteurisierung werden Kohlenhydrate und Aminosäuren, vorzugsweise 35-60 g Saccharose auf 100 g Lösung und 1-3 mol Glycin pro Liter Lösung sowie gegebenenfalls Calciumionen verwendet.

Es kann beispielsweise nach den deutschen Offenlegungsschriften 29 16 711 oder 32 37 512 gearbeitet werden.

Als Anionenaustauscher

Als Anionenaustauscher kommt vorzugsweise jedoch DEAE-Sepharose in Betracht.

Im Unterschied zu van Creveld und Baugh (s. früher) kommen diese Austauscher für die F VIII-Reinigung doch in Betracht, jedoch unter Bedingungen, wie sie bisher nicht bekannt waren und nachfolgend noch beschrieben werden.

Als wesentlich haben sich die Adsorptionsbedingungen erwiesen. Nämlich ebenfalls überraschend wurde gefunden, daß der F VIII im physiologischen Salzmilieu, bevorzugt bei pH 5,5, weitgehend selektiv an DEAE-®Sepharose gebunden wird, während Begleitproteine wie das Fibrinogen und Fibronectin im Überstand bleiben (Batch-Verfahren) oder eine Säule passieren. Schließlich wurde auch überraschend gefunden, daß eine pasteurisierte Lösung der Kryoglobuline überhaupt bei pH 5,5 im physiologischen Salzmilieu chromatographiert werden kann, da bei diesen Bedingungen bereits bestimmte Kryoglobuline präzipitieren, vor allem das Fibrinogen. Die Adsorption und speziell die Spezifität, mit der der F VIII an die Anionenaustauscher gebunden wird, nimmt zum Neutralpunkt hin stark ab. Unter normalen Chromatographiebedingungen jedoch wird DEAE erst oberhalb pH 7,0 beladen. Die Präzipitation bleibt in dem hier beschriebenen Verfahren jedoch aus, weil die Kohlenhydrate, die von der Pasteurisation her in der Kryolösung vorliegen, das Fibrinogen und Cig im leicht sauren Medium in Lösung halten.

Ein Vorteil des beschriebenen Verfahrens besteht also darin, daß bei der Anionenaustauscheradsorption von pasteurisiertem Kryoglobulin Fibrinogen und Fibronectin im Überstand oder Durchlauf bleiben und daraus als pasteurisierte Produkte gewonnen werden können. Fibronectin bespielsweise nach der deutschen Offenlegungsschrift 28 48 529.

Die Chromatographie beispielsweise an DEAE- oder QAE-Austauschern erfolgt im leicht sauren Milieu (pH 5,5) und an Austauschern, die entsprechend äquilibriert wurden, zum Beispiel mit 0,1 mol/l Na-Acetat-Puffer, der 0,1 mol/l Lysin enthielt. Mit diesem Puffer kann auch die pasteurisierte F VIII-haltige Lösung auf das doppelte Volumen verdünnt werden, bevor sie im Batch- oder Säulenverfahren mit dem Austauscher behandelt wird. Die Adsorption im Batch, die vorzugsweise verwendet wird, hat den Vorteil, daß man während der Adsorption die Bindung des F VIII an den Austauscher über die funktionelle Bestimmung des F VIII im Überstand verfolgen kann und nach Verschwinden der Aktivität die nicht adsorbierten Faktoren durch Sedimentation oder Zentrifugation vom lonenaustauscher abtrennen und unmittelbar danach mit der Waschung des F VIII-beladenen Austauschers beginnen kann. Während die Waschung vorteilhafterweise im Batch durchzuführen ist, beispielsweise auf einer Nutsche, empfiehlt sich für die Elution die Überführung des Austauschers in eine Säule, denn die säulenchromatographische Elution hat den Vorteil, daß der F VIII relativ konzentriert anfällt. Unter Versuchsbedingungen in der Größenordnung von 50 - 100 IE F VIII/ml.

Für die Waschung des mit F VIII beladenen Austauschers sind besonders solche Puffer geeignet, die einerseits den F VIII nicht ablösen, andererseits aber für eine möglichst quantitative Abtrennung unspezifischer Proteine wie der Immunglobuline und damit auch der Isoagglutinine geeignet sind. Als ein entsprechender Puffer erwies sich überraschenderweise ein Puffer, in welchem das Ausgangsmaterial zur Adsorption gelöst werden kann, und der 0,1 mol/l Na-Acetat, 0,1 mol/l Lysin und 1 g/l NaCl bei pH 5,5 enthält. Mit diesem Puffer wurde der F VIII beladene Ionenaustauscher so lange gewaschen, bis das Eluat frei von Isoagglutininen war. Für die Testung wurde der hochempfindliche Coombs-Test verwendet, der auch inkomplette Antikörper anzeigt.

Für die Desorption des F VIII von den Anionenaustauschern kommen konzentrierte Salzlösungen in Betracht, beispielsweise NaCl enthaltende.

Als vorteilhafter jedoch haben sich andere Salze von Halogenen mit Na, K oder Ca erwiesen: KBr, NaBr und CaCl₂. Sie haben den Vorteil, daß der F VIII als relativ scharfer Peak mit einer hohen Aktivität pro Volumen eluiert wird, ein Vorteil, der für die Fällung wichtig ist.

Die Konzentrationen liegen im Bereich von 0,05 mol/l bis zur Sättigungsgrenze.

Die Ausbeute hängt schließlich auch ganz wesentlich von der Elutionsgeschwindigkeit ab, die in einer Größenordnung von 1-10 ml/cm²/h, vorzugsweise 5-7 ml/cm²/h, sein sollte.

Das F VIII-haltige Eluat läßt sich mit den üblichen Methoden konzentrieren, nämlich durch die Fällung mit Neutralsalzen, wie Ammoniumsulfat oder NaCl, vorteilhafterweise mit NaCl, das im Gegensatz zu Ammonsulfat schneller ausdialysiert werden kann und auch nicht komplett entfernt werden muß.

Die Weiterverarbeitung der F VIII-haltigen Fällung erfolgt in der Weise, daß der Fällungsrückstand beispielsweise in einem Puffer pH 6,9, der Na-Citrat (0,02 mol/l), NaCl (0,06 mol/l), Glycin (20 g/l) und Albumin (5 g/l) enthält (Dialysepuffer), gelöst und gegen denselben Puffer ohne Albumin bis zum Gleichgewicht dialysiert wird. Die dialysierte Lösung wird durch Verdünnung mit dem albuminhaltigen Dialysepuffer auf eine Aktivität von 25 bis 30 IE F VIII/ml eingestellt und nach Sterilfiltration abgefüllt und gegebenenfalls gefriergetrocknet. Das Endpräparat ist als ein weißes Lyophilisat gekennzeichnet, das sich in weniger als einer Minute löst, eine F VIII-Aktivität von 5-10 IE/mg Protein besitzt, pasteurisiert und isoagglutininfrei ist. Gegenüber Produkten des Standes der Technik hat es den Vorteil, daß es frei von unerwünschten Proteinen ist und speziell solchen, die als Alloantigene zu einer Sensibilisierung führen könnten. Blutgruppenunverträglichkeiten wurden durch das Präparat nicht hervorgerufen. Eine Übertragung von Viruserkrankungen, speziell der verschiedenen Formen der Hepatitis, erscheint ausgeschlossen. Zu den Vorteilen des Verfahren gehört, daß es vergleichsweise einfach ist und deshalb problemlos in einen industriellen Maßstab übertragen werden kann und aus wenigen Arbeitsschritten besteht. Die geringe Zahl der Arbeitsschritte dokumentiert sich in der guten Ausbeute, denn erfahrungsgemäß ist jeder Reinigungschritt mit einem mehr oder weniger großen Aktivtätsverlust verknüpft.

F VIII kann nach folgendem Verfahren bestimmt werden: Ein Teil, zum Beispiel 0,1 ml partielles Thromboplastin, zum Beispiel hergestellt nach der deutschen Offenlegungsschrift 23 16 430 wird mit einem Teil F VIII-Mangelplasma und einem Teil verdünnten Normalplasma vermischt. Diese Mischung wird 6 Minuten bei 37° C gehalten. Nach Zusatz von einem Teil einer auf 37° C vorgewärmten 0,025 molaren Calciumchlorid-Lösung wird die Zeit bestimmt, die vom Zusatz der Calciumchlorid-Lösung bis zum Auftreten eines Gerinnsels verstreicht. Zur quantitativen Aussage wird eine mit einer Normalplasma-Verdünnungsreihe erstellte Eichkurve herangezogen.
1 Internationale Einheit (= 1 IE) F VIII entspricht der F VIII-Aktivität von 1 ml Normalplasma als Substandard zum 3. Internationalen WHO-Standard.

Im folgenden ist das Verfahren für die Gewinnung einer pasteurisierten und isoagglutininfreien F VIII-Präparation erläutert:

### Beispiel

### 1. Ausgangsmaterial

250 g Roh-Kryopräzipitat wurden in 750 ml einer NaCl-Lösung (0,08 mol/l), die Glycin (0,25 mol/l) und Heparin (1,25 USP-E/ml) enthielt, unter Erwärmen auf 30-37° C gelöst. Das ergab 1 000 ml Lösung mit einer Konzentration von 0,06 mol/l NaCl, 0,2 mol/l Glycin und ca. 1 USP-E Heparin/ml. Der pH-Wert der Lösung wurde mit 1 N HCl auf pH 6,5 eingestellt.

### 2. Aluminiumhydroxid-Adsorption

Zu 1 000 ml Lösung aus 1. wurden 80 ml einer 10 g/l Aluminiumhydroxid enthaltenden Suspension (Behringwerke Marburg) zugegeben und 15 min gerührt (Temperatur etwa 30°C). Danach wurde 15 min bei 3 000 x g zentrifugiert, der Rückstand verworfen und der Abguß nach Zusatz von Stabilisatoren pasteurisiert.

### 3. Pasteurisierung

1 000 ml Abguß aus 2. wurden in dieser Reihenfolge mit folgenden Stabilisatoren versetzt: 5 ml CaCl₂-Lösung, 1 mol/l (5 mmol/l) 1 000 g Saccharose (500 g/kg Lösung) 150 g Glycin (2 mol zu 1 l Lösung). Der pH-Wert wurde mit 2 N NaOH auf pH 7,3 eingestellt. Durch die Zusätze vergrößerte sich das Volumen auf 1 700 ml. Die Lösung wurde 10 Stunden bei 60° C im Wasserbad gehalten.

### 4. Ionenaustauscher-Behandlung

1 700 ml Lösung aus 3. wurden mit 1 700 ml einer Lösung, die 0,2 mol/l Na-Acetat, pH 5,5, und 0,2 mol/l Lysin enthielt, verdünnt. Der pH-Wert wurde mit verdünnter Essigsäure auf pH 5,5 eingestellt und 70 ml mit einer Lösung, enthaltend Na-Acetat (0,1 mol/l), pH 5,5, Lysin (0,1 mol/l) und NaCl (1 g/l), äquilibrierte DEAE-®Sepharose 6B Cl zugesetzt. Es wurde 2-3 Stunden bei Raumtemperatur gerührt und die Bindung des F VIII durch Bestimmung der Aktivität im Überstand bestimmt. Als die F VIII-Aktivität von 4 IE auf 0,1 IE/ml gefallen war, wurde das Adsorbens durch Zentrifugation abgetrennt.
Der Überstand wurde abgegossen und das Adsorbens vom Rest des Überstandes abgetrennt. Durch Waschen mit einer Lösung enthaltend 0,1 mol/l Na-Acetat, pH 5,5, 0,1 mol/l Lysin und 1 g/l NaCl wurde die Sepharose von eingeschlossenem Protein befreit und dann mit demselben Puffer in eine Säule eingeschlämmt (Maße: 10x3 cm).
In der Säule wurde der Austauscher so lange gewaschen, bis keine Lichtabsorption bei 280 nm mehr meßbar war und die Isoagglutininwerte mit dem Coombstest an der Nachweisgrenze lagen.

### 5. Elution

Der Austauscher aus 4. wurde mit einer 0,1 mol/l Na-Acetat, 0,1 mol/l Lysin und 0,3 mol/l CaCl₂ enthaltenden Lösung, pH 5,5, eluiert. Dabei erschien ein bei einer Wellenlänge von 280 nm meßbarer Peak. Die entsprechenden Fraktionen wurden gesammelt und ein Volumen von 180 ml mit 40 IE F VIII/ml erhalten.

### 6. Fällung von F VIII

180 ml Eluat aus 5. wurden mit 2,2 mol/l Glycin sowie 150 g/l NaCl versetzt und 30 min bei Raumtemperatur gerührt.
Die Fällung war an einer deutlichen Trübung erkennbar. Der Niederschlag wurde durch 30 min Zentrifugation bei 30 000 x g in der Ultrazentrifuge abgetrennt und nach Abgießen des Überstandes über Nacht bei 4° C gehalten.

### 7. Aufarbeitung

Der Niederschlag aus 6. wurde in 145 ml Puffer, pH 7,0, aufgenommen, der 0,02 mol/l Tri-Na-Citrat, 0,06 mol/l NaCl, 10 g/l Glycin und 5 g/l Human-Albumin (Lösepuffer) enthielt. Für die Lösung wurde eine Aktivität von 40 IE F VIII:C/ml bestimmt. Sie wurde 3 Std. bei Raumtemperatur gegen obigen Puffer, der kein Albumin enthielt, dialysiert, das Dialysat auf 30° C erwärmt und 30 min bei 30 000 x g und 25° C zentrifugiert. Nach einer F VIII-Bestimmung, die 36 IE/ml ergab, wurde die Lösung mit Lösepuffer auf 30 IE/ml eingestellt, die Lösung auf 37° C erwärmt und auf einem Membranfilter sterilfiltriert.
Es wurde in Fläschchen abgefüllt, eingefroren und lyophilisiert.

## Patentansprüche

1. Verfahren zur Herstellung einer Faktor VIII-Präparation, dadurch gekennzeichnet, daß eine pasteurisierte Faktor VIII enthaltende Lösung aus einem- Kryopräzipitat aus Humanplasma in Gegenwart eines Kohlenhydrats und einer Aminosäure mit einem DEAE-, QAE- oder Ecteola-gruppentragenden Austauscher auf der Basis von Cellulose, Sephadex® oder Sepharose® bei einem pH von 5 - 6,5 behandelt wird, der Austauscher gewaschen wird und der F VIII mit chaotropen Lösungen, vorzugsweise mit einer Lösung von CaCl₂ eluiert wird.

2. Verfahren zur Herstellung einer Faktor VIII-Präparation, dadurch gekennzeichnet daß Kryopräzipitat unter Zugabe von Saccharose und Glycin, vorzugsweise 35 - 60 g Saccharose/100 ml und 1-3 mol Glycin/l gelöst wird, daß Calciumionen vorzugsweise 1-20 mmol/l, zugesetzt werden, daß gegebenenfalls pasteurisiert abgekühlt und verdünnt wird, und daß bei pH 5 - 6,5 der F VIII:C an einen DEAE-, QAE- oder Ecteola-gruppentragenden Austauscher auf der Basis von Cellulose, Sephadex® oder Sepharose® adsorbiert und das Adsorbens gewaschen wird, bis es frei von Fremdproteinen, speziell Isoagglutinen, ist, und daß der F VIII:C durch Elution mit einer gepufferten konzentrierten chaotropen Salzlösung, vorzugsweise mit einer Lösung von CaCl₂ eluiert, dialysiert und lyophilisiert wird.

3. Verfahren nach Anspruch 1 oder 2, dadurch gekennzeichnet, daß man das Adsorbens mit einer gepufferten verdünnten Salzlösung wäscht.

4. Verfahren nach Anspruch 1 oder 2, dadurch gekennzeichnet, daß der Austauscher DEAE-Sepharose® ist.

## Claims

1. A process for the production of a factor VIII preparation, which comprises a pasteurized solution, containing factor VIII, of a cryoprecipitate of human plasma being treated with an exchanger which carries DEAE, QAE or Ecteola groups and is based on cellulose, Sephadex® or Sepharose® at a pH of 5 - 6.5 in the presence of a carbohydrate and of an amino acid, the exchanger being washed, and the F VIII being eluted with chaotropic solutions, preferably with a solution of CaCl₂.

2. A process for the production of a factor VIII preparation, which comprises cryoprecipitate being dissolved with the addition of sucrose and glycine, preferably 35 - 60 g of sucrose/100 ml and 1 - 3 mol of glycine/l, calcium ions, preferably 1 - 20 mmol/l, being added, where appropriate pasteurization, cooling and dilution being carried out and, at pH 5 - 6.5, the F VIII:C being adsorbed onto an exchanger which carries DEAE, QAE or Ecteola groups and is based on cellulose, Sephadex® or Sepharose®, and the adsorbent being washed until it is free of foreign proteins, in particular isoagglutinins, and the F VIII:C being eluted by elution with a buffered concentrated chaotropic salt solution, preferably with a solution of CaCl₂, and being dialyzed and freeze-dried.

3. The process as claimed in claim 1 or 2, wherein the adsorbent is washed with a buffered dilute salt solution.

4. The process as claimed in claim 1 or 2, wherein the exchanger is DEAE-Sepharose®.

## Revendications

1. Procédé de fabrication d'une composition de Facteur VIII, caractérisé en ce qu'une solution pasteurisée contenant du Facteur VIII provenant d'un cryoprécipité de plasma humain est traitée en présence d'un glucide et d'aminoacides avec un échangeur d'ions portant des groupes DEAE-, QAE- ou Ecteola- à base de cellulose, de Sephadex® ou de Sepharose® à un pH de 5 à 6,5, l'échangeur est lavé et le Facteur VIII est élué avec des solutions chaotropes, de préférence avec une solution de CaCl₂.

2. Procédé de fabrication d'une composition de Facteur VIII, caractérisé en ce que du cryoprécipité est dissous avec addition de saccharose et de glycine, de préférence 35 à 60 g de saccharose/100 ml et 1-3 moles de glycine/l, des ions calcium, de préférence 1-20 mmol/l, sont ajoutés, la solution est éventuellement pasteurisée, refroidie et diluée, le Facteur VIII:C est adsorbé à un pH de 5-6,5 sur un échangeur d'ions portant des groupes DEAE-, QAE- ou Ecteola- à base de cellulose, Sephadex® ou Sepharose ®, l'adsorbant est lavé jusqu'à ce qu'il soit dépourvu de protéines étrangères, en particulier d'isoagglutinines, puis le Facteur VIII:C est élué par élution avec une solution saline chaotrope concentrée tamponnée, de préférence une solution de CaCl₂, puis dialysé et lyophilisé.

3. Procédé selon la revendication 1 ou 2, caractérisé en ce qu'on lave l'adsorbant avec une solution saline diluée tamponnée.

4. Procédé selon la revendication 1 ou 2, caractérisé en ce que l'échangeur d'ions est du DEAE-Sepharose®.
